# EUROPEAN PATENT APPLICATION

(11) **EP 2 606 929 A1**
(43) Date of publication of application: **26.06.2013**
(21) Application number: 11194860.0
(22) Date of filing: 21.12.2011
(51) Int. Cl.: A61M 39/08, A61M 39/10, A61M 39/06

(54) **Catheter system**

(71) Applicant: Tradinco AB, 103 87 Stockholm (SE)
(72) Inventor: Liska, Jan, 114 43 Stockholm (SE)
(74) Representative: Mattsson, Niklas

(57) **Abstract**

The present invention provides a male luer connector for sealing a female luer connector, said male luer connector comprising a base portion adapted to removably connect with the female luer connector; a protruding portion adapted to be inserted into the female luer connector; a through hole extending through the protruding portion and the base portion and adapted to receive a needle; and a valve device arranged in said base portion and adapted to block fluid flowing in said through hole in the direction from the female luer connector via the protruding portion out of the male luer connector when the male luer connector is connected to the female luer connector. The present invention also provides a catheter hub comprising such a male luer connector as well as a method of inserting a catheter in the vascular system of a patient.

## Description

### Technical Field of the Invention

The present invention relates to a male luer connector and a catheter hub used when inserting a catheter into the vascular system of a patient.

### Background Art

A typical catheter used for peripheral access to the vascular system comprises of a plastic tubular member attached to a distal end of a catheter hub which also has an open proximal end with a female luer connection. Prior to insertion the catheter also holds a steel puncture needle for penetrating the skin, underlying tissues and finally the vasculature. Once the needle point is inside the vessel the plastic tubular member is pushed over the puncture needle further into the vessel. The next step is the removal of the steel puncture needle from the catheter throughout the proximal female luer connection, and once removed the female luer opening is sealed by a separate solid male luer cap. However, when removing the puncture needle the distal part of the plastic tubular member positioned in the vessel must be compressed from the outside by pressing a finger over the skin at the distal end, to prevent blood from leaking out from the open proximal female luer, until it is sealed by the separate luer cap.

It is often difficult to find and compress the distal opening and therefore it is quite often a leakage of blood spill through the proximal female luer connect until a luer cap has been applied. The blood spill exerts a certain risk for blood mediated infection from the patient to hospital staff. The open end of the catheter hub is also exposed for external contamination until it is sealed by the separate luer cap.

To summarize, there is a need in the art for improved methods and systems for inserting a catheter into the vascular system of a patient.

### Summary of the Invention

It is an object/aim of the present invention to provide a system that overcomes at least some of the problems encountered in the prior art.

As a first aspect of the invention, there is provided a male luer connector for sealing a female luer connector, the male luer connector comprising
- a base portion adapted to removably connect with the female luer connector;
- a protruding portion adapted to be inserted into the female luer connector;
- a through hole extending through the protruding portion and the base portion and adapted to receive a needle; and
- a valve device arranged in the base portion and adapted to block fluid flowing in the through hole in the direction from the female luer connector via the protruding portion out of the male luer connector when the male luer connector is connected to the female luer connector.

A "male luer connector" refers to a male part in the standardized system of small-scale fittings known as Luer taper, known to the skilled person. The Luer Taper may for example be those connectors that are defined in the ISO 594 standards. The Luer taper connections may both be Luer-Lock and Luer-Slip connections, known to the skilled person. A male luer connector is thus adapted to mate with a female luer connector and a female luer connector is thus adapted for receiving and mating with a male-taper fitting, thereby creating a leak-free connection.

A base portion refers to the "body" of the luer connector. The luer connector has also a protruding portion, adapted to be inserted into the female luer connector, such as into the tube or a portion of the female luer connector.

The male luer connector may thus removably connect with a connectable portion of the female luer connector.

A "through hole" refers to a through passage or channel that extends in the luer connector from the protruding portion to the base portion and to the exterior of the luer connector. The through hole may thus be a central channel. The through hole is adapted to receive needle, such as a puncture needle, and may have inner dimensions adapted to the outer dimensions of a puncture needle, such that dead space is minimized. A "puncture needle" refers to a needle adapted for puncturing the vascular system of a patient, such as an artery or vein. A puncture needle held by the cap may thus extend to the outside from both the protruding portion and from the base portion.

The through hole may be adapted to substantially frictionless receive a needle, which means that a needle may be inserted in the through hole almost with no friction. This is advantageous if for example the needle is used for inserting a catheter into the vascular system of a patient through the luer connector. Usually, when a catheter is pushed over the needle used for puncturing the blood vessel and further into the vessel, there is a high risk of accidentally further perforating the vessel with the needle. However, with no or little friction, the risk of dislocating the needle such that it accidentally punctures other parts of the vessel or surrounding tissue as the catheter is pushed over the needle and into the vessel decreases. This means that if the through hole is adapted to receive the needle almost frictionless, the risk of inflammation, such as thrombothlebitis, decreases.

A "valve device" refers to any type of valve with capacity of restricting flow of fluid, such as liquid, in the through channel in the direction from the protruding portion to the outside. The valve device is thus adapted to receive a needle as a needle is inserted into the through hole.

The valve device may be adapted to withstand a specific pressure (a cracking pressure), such as the pressure of the back-flow of blood experienced in the through hole upon removal of a puncture needle if the luer cap is us in a catheter hub

The inventor has found that a luer connector according to the first aspect of the invention is advantageous for use e.g. when inserting a peripheral intravenous catheter into the vascular system of the patient. The luer connector may be provided on the catheter hub or in on a needle holder prior to insertion, thus omitting the procedure of applying a separate cap after withdrawing the needle from the patient, since the luer connector may function as a cap. This means that blood spill from the catheter may be eliminated at insertion thus reducing the risk for blood mediated infections.

The protruding portion of the connector may thus be regarded as a distal end if for example the male luer connector is attached to a catheter hub. The through hole may thus be a central channel extending from the distal end, through the base portion and out to the exterior of the luer connector.

In embodiments of the first aspect, the valve device is adapted to receive the needle in a substantially frictionless manner and/or adapted to allow a received needle to slide in the through hole in a substantially frictionless manner.

The valve device may be adapted to receive a needle almost or substantially frictionless. The valve device may also be adapted to allow a needle to slide with almost no friction in the through hole of the luer connector. As discussed above, this is advantageous if for example the needle is used for inserting a catheter into the vascular system of a patient through the luer connector. With no or little friction, the risk of dislocating the needle such that it accidentally punctures other parts of the vessel or surrounding tissue as the catheter is pushed over the needle and into the vessel decreases. Thus, if the valve device is adapted to receive the needle almost frictionless, the risk of inflammation, such as thrombothlebitis, decreases.

In embodiments of the first aspect, the valve device is adapted to seal against the needle when a needle is inserted in the through hole.

This may thus further decrease the dead volume in the through hole when a needle is present in the luer connector.

In embodiments of the first aspect, the protruding portion is adapted to be inserted into the female luer connector by means of sliding.

In embodiments of the first aspect, the protruding portion is threaded and thereby adapted to be inserted into the female luer connector by means of screwing.

Thus, the protruding portion may be adapted to slide into a receiving portion of the female luer connector, and/or it may be adapted to be screwed into the connecting portion of the female luer connector.

In embodiments of the first aspect, the valve device is a one way check valve, such as a duck-bill check valve.

A check valve (also known as a clack valve, non-return valve or one-way valve) refers to a valve, which normally allows fluid (liquid or gas) to flow through it in only one direction. The check valve may have two openings, one for fluid to enter and one for fluid to leave the check valve. The check valve may for example be made of a nonporous material, and it may be of a plastic or a metal. As an example, the check valve may comprise silicon.

As an example, the check valve may be adapted for receiving a needle in one direction and adapted for preventing fluid from flowing in the opposite direction.

The inventor has found that a check valve is suitable for a valve device in the male luer connector.

The check valve may be a duck-bill valve, which refers to a valve shaped like the beak of a duck. It may have one open end and the other end, the duckbill, having a flattened shape. Duckbill check valves allow free flow with positive differential pressure. With negative differential pressure, backflow is checked. When a fluid is pumped with enough pressure from the open end, the flattened end may open to permit the pressurized fluid to pass. When pressure is removed, however, the duckbill end returns to its flattened shape, preventing backflow. The duck-bill check valve may be made of rubber or a synthetic elastomer. As an example, the duck-bill check valve may comprise silicon.

The inventor has found that a one-way valve, such as a duck-bill check valve, is suitable for both receiving a needle from one direction and for blocking fluid flowing in the through hole in the opposite direction.

Further, the inventor has found that a dome slit valve may be used together with a one-way check valve. Therefore, in embodiments of the first aspect, the male luer connector is further comprising a dome slit valve arranged in the through passage with the convex surface facing the exterior when the male luer connector is connected to the female luer connector.

A dome slit valve refers to a valve having a convex/concave surface, shape substantially like a dome, i.e. substantially as a hemisphere. A slit is provided in the surface of the dome, and the dome slit valve is adapted to block fluid flowing in the direction from outside the convex surface to the other side of the dome, i.e. towards the center of the dome. The slit may be adapted to receive a needle, such as a puncture needle.

The use of a dome slit valve with the with the convex surface facing the exterior when the male luer connector is connected to the female luer connector prevents external contamination from the outside of the luer connector into the male luer connector and hence also into the female luer connector.

The dome slit valve may for example be made of or comprise an elastic material. The dome slit valve may for example comprise a polymer such as silicon.

The inventor has also found that a dome slit valve may be used alone as the valve device. This means that in embodiments of the first aspect, the valve device is a dome slit valve arranged in the through passage with the convex surface facing the exterior when the male luer connector is connected to the female luer connector.

As an example, a dome slit valve may be adapted to withstand a pressure from the concave surface which is the pressure of the back-flow of blood experienced in the through hole upon removal of a puncture needle if the luer cap is us in a catheter hub. The pressure may for example be the venous pressure or the arterial pressure.

As an example, the male luer connector may comprise a second dome slit valve arranged in the through passage with the convex surface facing female luer connector when the male luer connector is connected to the female luer connector.

Thus, two dome slit valves may be used in conjunction with opposite flow directions. This allows for both preventing external contamination from the outside of the male luer connector and preventing back-flow of blood from a catheter hub, if the male luer connector is used on a catheter hub.

According to the present invention, the slit edges of the dome slit valve may extend towards the center of the dome, thereby forming a tight passage extending from the surface of the dome towards the center of the dome.

Thus, the surface of the dome may, at the location of the slit, bend "inwards" towards the center of the dome, i.e. towards the center of the substantially hemisphere formed by the convex surface. This forms a tight passage, i.e. the extending edges seal against each other as they extend into the interior, or center, of the hemisphere formed by the convex surface. Thus, the slit extends towards the center of the hemisphere formed by the convex surface. The slit may for example extend the whole way to a fictional center of the hemisphere formed by the dome, or it may for example extend about halfway or about quarter of the way towards the center. This means that the opening is located closer to the center of the hemisphere formed by the convex surface. The extended edges forming a passage thus forms a type of duck-bill valve, further blocking fluid flowing from the concave surface through the slit out of the convex surface. The extended surfaces thus forms a lip-structure.

The formed passage may be adapted for receiving a puncture needle.

The inventor has found that a dome slit valve, in which the slit edges of the dome slit valve extend towards the center of the dome, thereby forming a tight passage extending from the surface of the dome towards the center of the dome, combines the properties of a duck-bill valve with the properties of a dome-slit valve. This means that when used on e.g. a catheter hub (such as on the proximal end of a catheter hub), it may both block fluid flowing from the catheter hub (such as backflow of blood) to the outside of the catheter hub as well as preventing external contamination from the outside into the catheter hub. Thus, such a "double valve concept" reduces the risk for external contamination/air to the inside of the cap or catheter hub/vasculature.

Further, according to the present invention, a dome slit valve may comprise at least one slit edge reinforcement means arranged on the concave side of the dome, the reinforcement means extending substantially perpendicular to the direction of the slit from at least one extended slit edge to the concave surface of the dome, for increasing the pressure the extended slit edges may withhold before opening.

The dome slit valve may for example be of silicon and the slit edge reinforcement means may be of silicon. Such a reinforcement means thus further restrict the flow of liquid through the slit. The reinforcement means may thus be located in the interior of the hemisphere formed by the dome, and thus be located on the side of the dome slit valve that faces the female luer connector when the male luer connector is attached to a female luer connector. The reinforcement means is thus be adapted to increase the pressure the slit of the dome may withstand, i.e. making it more "difficult" for a slit to open. If the dome slit valve comprises slit edges that extend towards the center of the dome, as described above, the reinforcement means, e.g. in the form of strips having about the same thickness as the extended slit edges, may extend perpendicularly from both of the extended slit edges towards the concave dome surface and be attached to the concave dome surface. The reinforcement means may extend from the tight passage formed by the extended slit edges towards the concave surface of the dome slit valve. Thus, the slit edges and the reinforcement means may form a X-shape when viewed from the direction of the concave surface.

In embodiments of the first aspect, the valve device is a shutter arranged in a coring-out channel of the base portion, wherein the channel extends from the through hole towards the outside of the luer cap, and wherein the shutter is arranged to seal the through hole in the absence of the needle.

The valve device may thus act as a mechanical shutter that seals the through hole due to movement of the shutter upon removal of a puncture needle. The shutter may reside in a coring-out channel in the base portion of the luer cap. The coring out channel may extend substantially perpendicularly compared to the through hole, and may extend all the way to the outside of the luer cap. The shutter may for example be made of metal.

The use of a valve device in the form of a shutter may for example facilitate sterilization of the valve device, since the valve device may comprise or consist of e.g. a metal.

Further, the shutter may be adapted to be spring loaded in the channel by the needle such that the shutter automatically seals the through hole in the absence of the needle.

Thus, the shutter may be spring-loaded a needle present in the through hole of the luer connector, such that the needle "push" the shutter from the through hole, e.g. during insertion of the needle. This allows for an automatic closing of the shutter upon removal of the needle due to the action of a sprung force that moves the shutter in the coring-out channel such that the through hole is sealed.

The shutter may for example be spring-loaded by means of a rubber band strung around the base portion of the luer cap. The rubber band may for example be made of silicon. The shutter may also be "internally spring loaded" i.e. it may comprise a flexible plastic that may be deformed as the needle is present in the through hole. Upon removal of the needle, the flexible plastic shutter may return to its original shape and thereby closing the through hole.

It is also to be understood that the through hole may be manually sealed by the shutter.

Further, if the valve device is a shutter, the through hole may comprise a removable inner tubing or casing in the base portion such that the shutter in the coring out channel is prevented from abutting a puncture needle present in the through hole. Thus, the shutter then instead abuts the tubing or casing. This may further reduce the friction between the puncture needle and the shutter.

The removable inner tubing or casing may for example comprise silicon.

In embodiments of the first aspect, the male luer connector is further comprising a cover to prevent contamination from the outside into the through hole when the male luer connector when connected to the female luer connector.

As a second aspect of the invention, there is provided the use of a luer connector according the first aspect in a catheter hub for restricting back flow of blood from the catheter to the exterior upon removal of a puncture needle from the catheter hub, wherein the luer connector is removably connected to a proximal female luer connector of the catheter hub and wherein the needle is the needle used to insert the catheter into the vascular system of a patient and is removed from the vascular system and the catheter hub through the through hole of the luer connector.

As a third aspect of the invention, there is provided a catheter hub comprising a catheter at a distal end, a female luer connector at a proximal end, a male luer connector according to the first aspect of the invention removably connected to the female luer connector, and a puncture needle extending through the catheter, the catheter hub and the through hole of the luer connector.

The terms and definitions used in the second and third aspect of the invention are as defined in connection with the other aspects of the invention hereinabove.

According to the present disclosure, the distal-proximal ends of a catheter hub refer to the flow direction in the catheter, as known to the skilled person. Thus, when attached to the body, the proximal part is thus the part farthest away from the body and the distal part is the part nearest the body.

Thus, the needle is arranged in the catheter hub with the tip of the needle at the distal end.

The use of the disclosed luer connector in a catheter hub according to the second aspect of the invention and the catheter hub according to the third aspect thus prevent leakage of blood spill through the proximal female luer connector, since the cap (i.e. the luer connector) is already in place as the needle is inserted. Consequently, a catheter hub according to the third aspect provides for a catheter assembly that seals the female luer connection from external contamination and leakage when the puncture needle is removed after insertion of a catheter, by already having the connector according to the first aspect of the invention attached to the proximal end as the catheter is inserted. Thus, the use of a luer connector according to the present disclosure allows for a one-hand operation when inserting a needle, and removes the step of compressing the distal opening of the catheter hub and attaching a separate cap or cover. Hence, the risk for blood mediated infection from the patient to hospital staff is decreased. Further, upon removal of the needle, the catheter hub is not exposed for external contamination since it is already "sealed" prior to insertion.

In embodiments of the third aspect, the catheter hub comprises a shutter as described above and wherein
the through hole of the male luer connector ends with a removable hollow casing for the needle in the base portion, the casing comprising an inlet facing the outside of the male luer connector and an outlet facing the female luer connector when the male luer connector is connected to the female luer connector, and a hollow interior between the inlet and outlet through which the needle extends, wherein the hollow casing is arranged such that the shutter abuts the casing and not the needle,
and the needle comprises a lock portion arranged between the tip and the catheter hub, the lock portion having a larger diameter in at least one direction compared to the diameter of the part of the needle extending through the catheter hub and the male luer connector,
and wherein the diameter of the through hole of the male luer connector, the diameter of the outlet of the casing and the diameter of the hollow interior of the casing are larger than the diameter of the lock portion of the needle, and wherein the diameter of the inlet of the hollow casing is smaller than the diameter of the lock portion of the needle such that the lock portion and the tip of the needle get stuck in the hollow casing upon removal of the needle from the catheter hub in the direction from the female luer connector to the male luer connector, such that the hollow casing may be withdrawn from the male luer connector together with the needle.

Thus, a removable hollow casing may be positioned at the end of the through hole such that a shutter, e.g. a spring-loaded shutter, abuts the casing instead of the needle. The hollow casing may thus surround the needle and the shutter may be spring loaded by the presence of the hollow casing in the through hole. This may further reduce the friction with which the needle may slide through the catheter hub. The needle has further a wider portion, the "lock portion" close to the tip of the needle, i.e. on the distal end compared to the catheter hub. This may be a flattened portion of the needle such that the lock portion has a larger diameter in one direction. Further, the hollow casing as described above provides for automatic needle tip protection after removal from of the needle from the catheter hub, which thus prevents the medical practitioner from the tip needle. Thus, as understood from the dimensions of the needle, the through hole and the hollow casing, the needle may slide through the casing when removed from the catheter until the lock portion, having a smaller diameter than the inlet of the casing, abuts the inlet of the casing, i.e. the proximal end of the casing. Since the casing is removably connected to the base portion, the casing may be removed when the lock portion is stuck within the casing as the needle is further removed in the direction from the distal end to the proximal end of the catheter hub. Upon removal of the needle and hence the casing, the shutter, such as a spring loaded shutter, may seal the through hole and prevent blood from spilling out of the catheter. Thus, the use of a casing and a shutter as described above allows for automatic closing of the through hole as well as an automatic tip protection of the needle upon removal of the needle from the catheter hub.

To further secure the tip within the casing upon removal of the needle from the catheter hub, the outlet, i.e. the "distal end" of the casing may be provided with automatic locking means to seal the tip within the hollow casing. The automatic locking means may be arranged to seal the outlet of the casing upon removal of the casing from the male luer connector.

In embodiments of the second or third aspect, a needle holder is removably connected to the male luer connector.

Thus, such a needle holder further provides stability for the needle of the catheter hub upon insertion of a catheter into the vascular system, and further provides for a one-hand operation when inserting and removing the needle. Since the needle holder is removably connected to the male luer connector, it may removed from the male luer connector.

In embodiments of the third aspect, the external needle holder comprises a valve device for blocking fluid flowing from the needle holder into the through hole of the male luer connector.

Such a valve device may for example be the means discussed in relation to the first aspect above, such as a one-way duck bill valve or a dome slit valve.

As a fourth aspect of the invention, there is provided a method for inserting a catheter in the vascular system of a patient, comprising the steps of:
a) providing a catheter hub according to any one of claims 14-16,
b) puncturing a vessel of the vascular system of the patient with the puncture needle and pushing the catheter over the puncture needle into the vessel; and
c) pulling the puncture needle out from the vascular system and the catheter hub via the male luer connector.

A method for inserting the catheter according to the fourth aspect decreases the risk of blood flowing back through the catheter and leaking out of the catheter hub, thus decreasing the risk of blood mediated infection from the patient to hospital staff. The method further reduces the risk of external contamination into the catheter hub. Further, the method decreases the risk for blood mediated infection from the patient to hospital staff upon insertion of a catheter.

In embodiments of the fourth aspect, the method is further comprising the step of
d) putting a cover on the male luer connector for preventing the risk of external contamination into the catheter hub.

This may thus further decrease the risk of external contamination into the catheter hub.

### Brief description of the drawings

Figure 1 shows a male luer connector according to the present disclosure having a duck-bill valve as a valve device.
Figure 2 shows a male luer connector according to the present disclosure having a duck-bill valve and a dome slit valve.
Figure 3a and 3b shows a top and bottom view, respectively, of the luer connector of Fig. 1.
Figure 4 shows the male luer connector of Fig. 1 having a puncture needle and an external needle holder.
Figure 5 shows a dome-slit valve in which the edges of the slit extend into the center of the hemisphere formed by the dome.
Figure 6 shows the dome slit valve of Fig. 5 arranged in a male luer connector.
Figure 7 shows a male luer connector according to the present disclosure attached to a catheter hub.
Figure 8a-c show a schematic example of a male luer connector in which the valve device is a spring loaded shutter.
Figure 9a-c show a further schematic example of a male luer connector in which the valve device is a spring loaded shutter.
Figure 10a-c show a schematic example of a male luer connector in which a needle is present. The male luer connector comprises a hollow casing that protects the tip of the needle upon removal from the male luer connector and has a shutter that automatically closes the through hole when the needle has been removed.

### Detailed description of the Invention

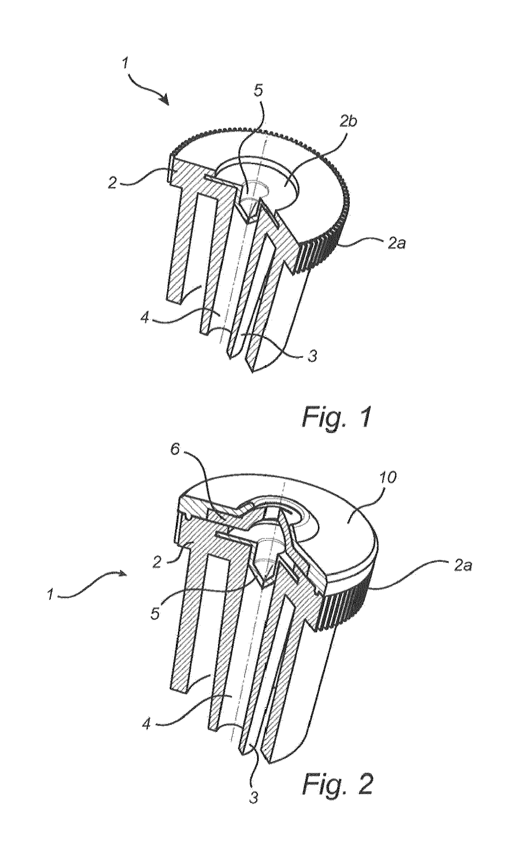

Fig. 1 shows an embodiment of a male luer connector 1 according to the present invention. The male luer connector 1 has a base portion 2 adapted to removably connect with a connectable portion of a female luer connector. The base portion 2 has a corrugated part 2a, that further facilitates handling, such as screwing of the male connector, when connecting the male luer connector 1 to a female luer connector. The base portion 2 also has a relief structure 2b for holding a valve device 5. The male luer connector 1 further comprises a protruding portion 3 adapted to be inserted into a tube or portion of the female luer connector, a through hole 4 extending through the protruding portion 3 and the base portion 2 and adapted to frictionless receive a needle. A valve device 5 is arranged in the base portion 2 and is adapted to block fluid flowing in the through hole 4 in the direction from the female luer connector via the protruding portion 3 out of the male luer connector 1 when the male luer connector 1 is connected to the female luer connector. The valve device 5 is in this embodiment a one-way duck-bill valve, and a needle (not shown) may be inserted or enter the through hole 4 or channel through the duck-bill valve from the end outside the base portion 2 and continue to the outside of the male luer connector 1.

The connector 1 may thus function as a cap on a catheter hub, if connected to an open female part of a catheter hub. In other words, the connector 1 may thus be described as cap having a central channel extending from a proximal opening of the cap through the whole extension of the male luer part of the cap and with a distal opening. The channel contains a one-way valve with a flow direction from the proximal to the distal end of the luer cap, and by that means preventing back flow through the luer cap from the distal opening to the proximal end of the male luer when the puncture needle is released from the catheter as well as after the puncture needle has been removed.

Fig. 2 shows a similar luer connector 1 as shown in Fig. 1, but also having a dome slit valve 6 arranged in the base portion 2. The duck-bill valve 5 is arranged and positioned closer to the end of the protruding portion 3 of the luer connector 1 in the through hole 4 compared to the dome slit valve 6, such that the duck-bill valve 5 is closer to the female luer connector when the male luer connector 1 is connected to a female luer connector. The connector 1 further contains a locking plate 10 for fixing the dome-slit valve on the base portion 2 of the luer connector 1. The dome slit valve 6 is arranged such that the convex surface faces the exterior of the male luer connector 1 when connected to a female luer connector. In this embodiment, a needle (not shown) that is inserted into the through hole 4 from outside the base portion 2 first passes the slit of the dome slit valve 6 and then the duck-bill valve 5 and then continues through the protruding portion 3. Both the dome-slit valve 6 and the duck-bill valve 5 are arranged for receiving a needle almost frictionless, i.e. the needle may glide smoothly through the luer connector 1 through the through hole 4 if inserted from the base portion 2 of the male luer connector 1.

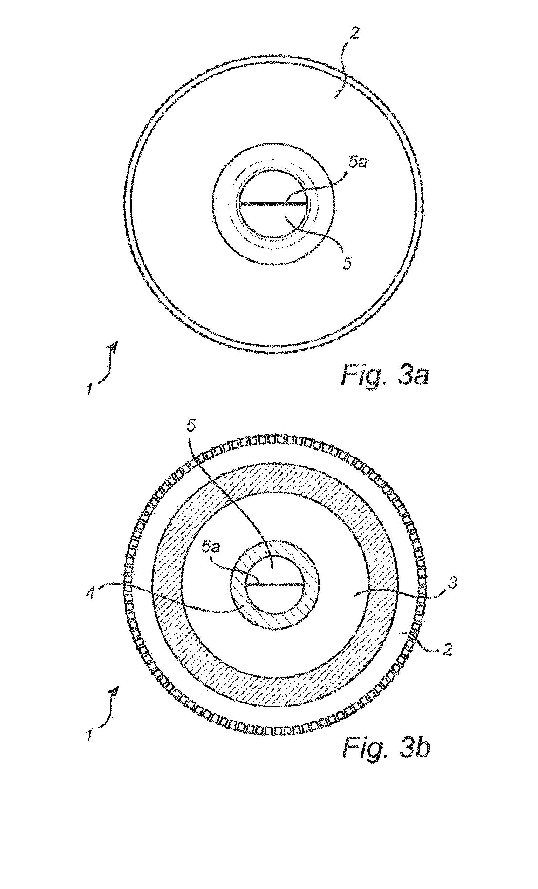

Fig. 3a shows a top view of the luer connector of Fig. 1, i.e. when viewed from the direction of the base portion 2. The valve device 5, in the form of a one-way duckbill valve is seen having a slit 5a. A needle may be inserted substantially frictionless into the through hole 4 via the duck-bill valve from the "top" of the luer connector shown in Fig. 3a.

Fig. 3b shows a bottom view of the luer connector of Fig. 1 when viewed from the direction of the protruding portion 3. This side of the male luer connector 1 is thus inserted into a connecting part of a female luer connector.

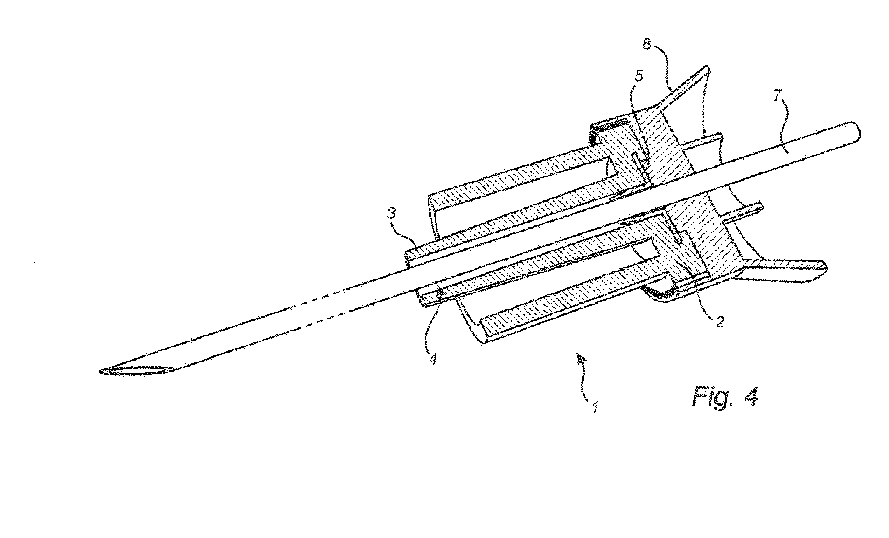

Fig. 4 shows a luer connector according to Fig. 1 having a puncture needle 7 inserted in the through hole 4. As seen in this figure, the diameter of the through hole 4 is only slightly larger than the puncture needle 7, in order to minimize dead volume. Further, the duck bill valve 5 seal tight against the needle 7 in the through hole 4. Fig. 4 also shows an external needle holder 8 for holding the needle 7 in position, i.e. for further stabilizing the position of the needle 7 during e.g. when using the needle 7 for inserting a catheter into the vascular system of a patient.

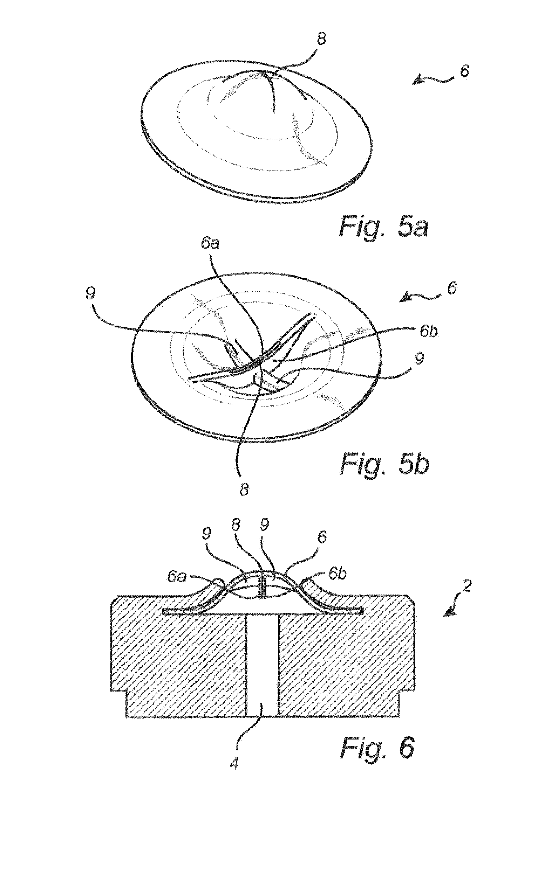

Fig. 5a and 5b shows a dome-slit valve 6 according to an embodiment of the present disclosure. The dome slit valve 6 has a convex surface, which forms a hemispherical structure, and a slit 8 in the surface of the dome.. In this embodiment, the edges 6a and 6b of the slit 8 extend into the formed dome, i.e. towards the center of the hemispherical structure formed by the dome. Thus, the extended edges 6a and 6b form a tight passage for a needle when a needle is passed through the slit 8. The edges 6a and 6b of the slit 8 thus forms a duck-bill function within the dome slit valve 6. The edges 6a and 6b may extend e.g. halfway towards the center of the hemispherical structure formed by the convex surface. The extended slit edges 6a and 6b of the dome slit valve 6, further comprises two slit edge reinforcement means 9 on the concave side of the dome slit valve 6. The reinforcement means 9 are in the form of two strips 9, extending in a direction that is perpendicular compared to the direction of the slit, as seen in Fig. 5b. The reinforcement means extend from the tight passage formed by the slit edges 6a and 6b and are attached on the "interior" surface of the hemisphere formed by the dome, i.e. the surface on the concave side of the dome slit valve 6. In this embodiment, the reinforcement strips 9 have a thickness that is about the same as the thickness of the slit edges 6a and 6b.

Fig. 6 shows the dome slit valve 6 of fig 5 when arranged in the base portion 2 of the luer cap. As seen from this figure, the convex surface is arranged such that it faces the exterior of the luer connector 1 when the male luer connector 1 is inserted into a female luer connector. The edges 6a and 6b thus extend into the "interior" of the dome when viewed from the exterior of the male luer cap. The reinforcement means 9 are attached to the extended slit edges such that e.g. half of the length of the extended slit edges are attached to the reinforcement means, as seen in Fig. 6.

A dome valve as shown in Figs. 5 and 6 thus has a double function. First of all, it prevents fluid flowing in the through hole 4 in the direction from the female luer connector via the protruding portion 3 out of the male luer connector 1 when the male luer connector 1 is connected to the female luer connector. This function is enhanced by the edges 6a and 6b extending towards the center of the dome, i.e. these edges function as a duck bill valve. Secondly, the dome slit valve prevents contamination from the outside into the through hole 4 when the male luer connector 1 is connected to a female luer connector.

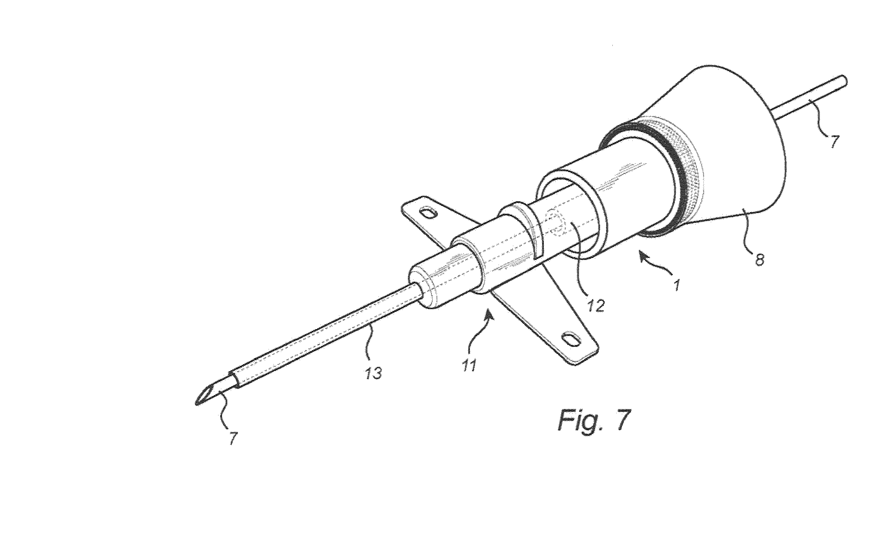

Figure 7 shows a catheter hub 11, or catheter assembly, according to the present disclosure. The catheter hub has a proximal female connector 12, to which a male luer connector 1 according to the present invention is connected. The male luer connector 1 thus functions as a luer cap on the catheter hub 11. A puncture needle 7 extends through the catheter hub 11 and through a catheter 13 that is fastened at a distal end of the catheter hub 11. A needle holder 8 is connected to the male luer connector 1 for holding the puncture needle 7 and thereby further stabilizing the position of the needle 7. The needle holder 8 is removably connected to the male luer connector 1. Further, as can be seen in this figure, the needle 7 further extends from the male luer connector 1 through the needle holder 8 from the end of the male luer connector 1 that is not facing the female luer connector 12. The needle 7 thus extend to the other side of the needle holder 8.

Thus, the male luer connector 1 together with the needle holder 8 is connected to the female luer connector 12 of the catheter hub 11 prior the insertion of the catheter 13 into the vasculature. The luer cap 1 and needle holder 8 thus holds the puncture needle 7 prior to insertion. Once the needle point is in the vessel and the catheter 13 is pushed over the puncture needle 7 into the vessel, the puncture needle 7 can be removed without the need for compressing the distal catheter end, because of the back flow valve (the valve device) inside the luer cap 1 prevents blood spill, thus reducing the risk for blood mediating infections. The inside of the catheter hub 11 is maintained sterile since there is no need for applying a separate luer cap to seal the proximal opening female luer 12. As discussed above, to further prevent external contamination or air to the interior of the catheter hub 11 due to a negative pressure inside the vasculature, which may cause the one-way valve in the luer cap to open, an additional valve in the luer cap with a flow direction opposite of the first valve may be used. An additional valve may also aid in keeping the inside of the luer connector and catheter hub 11 sterile, preventing contamination of external positive pressure as well as mechanical stress. Such a valve may be a dome-slit valve. However, if a dome slit valve as discussed in relation to Fig. 5 above is used, the need for an additional valve may be reduced, since such a dome slit valve, in which the edges of the slit extend into the interior of the dome, has a double-valve function as discussed above.

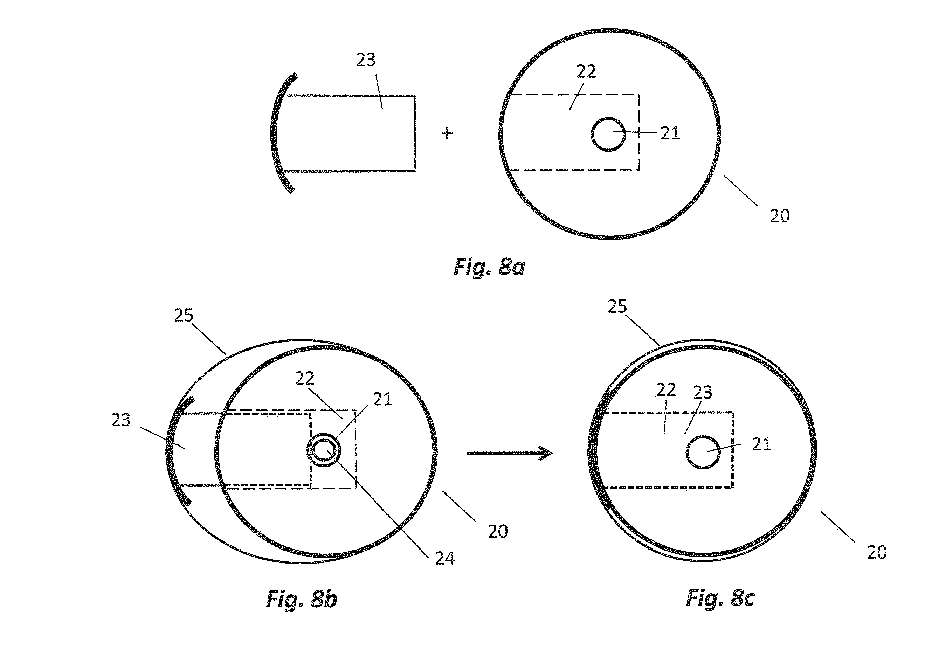

Figure 8a-c schematically show an example of a male luer connector having a shutter to seal the through hole. Fig. 8a shows a top view of the base portion 20 of the male luer connector. The through hole 21 is located substantially in the center of the base portion 20. The base portion 20 has a coring-out channel 22 extending from the through hole to the outside of the base portion 20. A shutter 23 may be inserted in the coring-out channel 22 to seal the through hole 21. Figure 8b shows the shutter 23 mounted in the coring out channel 22 by means of a silicon rubber band 25 extending around the base portion 20. In Fig. 8b, a 24 needle is present in the through hole 21, and the shutter 23 is "pushed back" by the needle and spring loaded due to the rubber band 25, The shutter 23 is prevented from sliding all the way into the coring out channel 22 and is thus prevented from sealing the through hole 21 in the presence of needle 24 in through hole 21. When the needle 24 is removed, the shutter 23 may slide all the way in the coring out channel 22 and seal the through hole 21 due to the action of the rubber band 25, i.e. the sprung force of the rubber band 25, as seen in Fig. 8c.

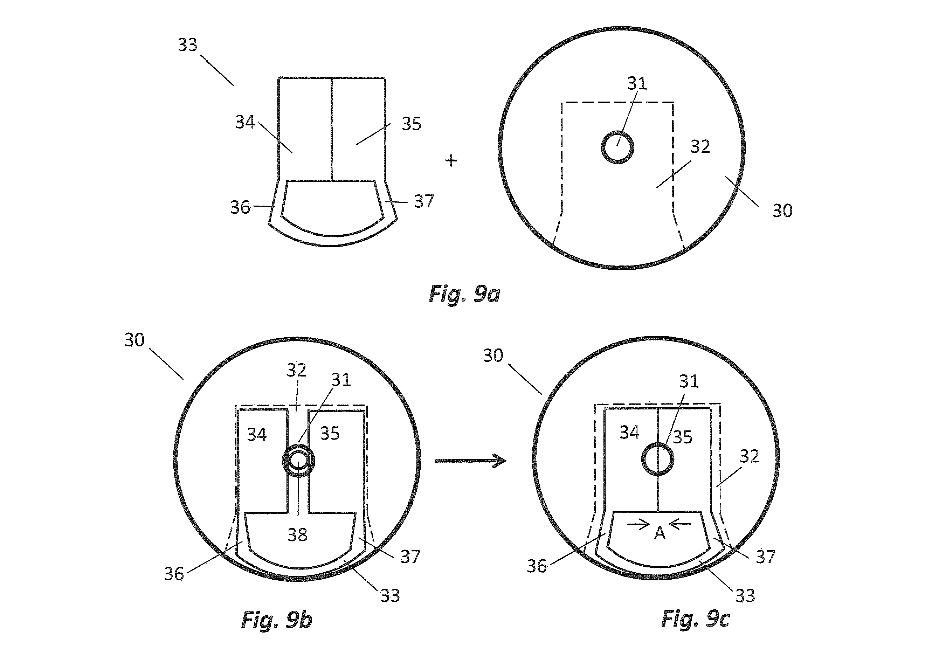

Figure 9a-c schematically show a further example of a male luer connector having a shutter to seal the through hole. Fig. 9a shows a top view of the base portion 30 of the male luer connector. The through hole 31 is located substantially in the center of the base portion 30. The base portion 30 has a coring-out channel 32 extending from the through hole to the outside of the base portion 30. A shutter 33 may be inserted in the coring-out channel 32. The shutter 33 comprises two sealing members 34 and 35 connected by arms 36 and 37. The shutter 33 is made of an elastic material, such as an elastic plastic. This means that when a needle 38 is present in the through hole 32, as shown in Fig. 9b, the sealing members 34 and 35 are separated by the needle 38, which thus prevents the sealing members 34 and 35 from sealing the through hole 31. Upon removal of the needle 38, the sealing members 34 and 35 are forced to abut each other due to the elastic action of the arms 36 and 37 in the directions indicted by arrows A, as seen in Fig. 9c, thereby sealing the through hole 31.

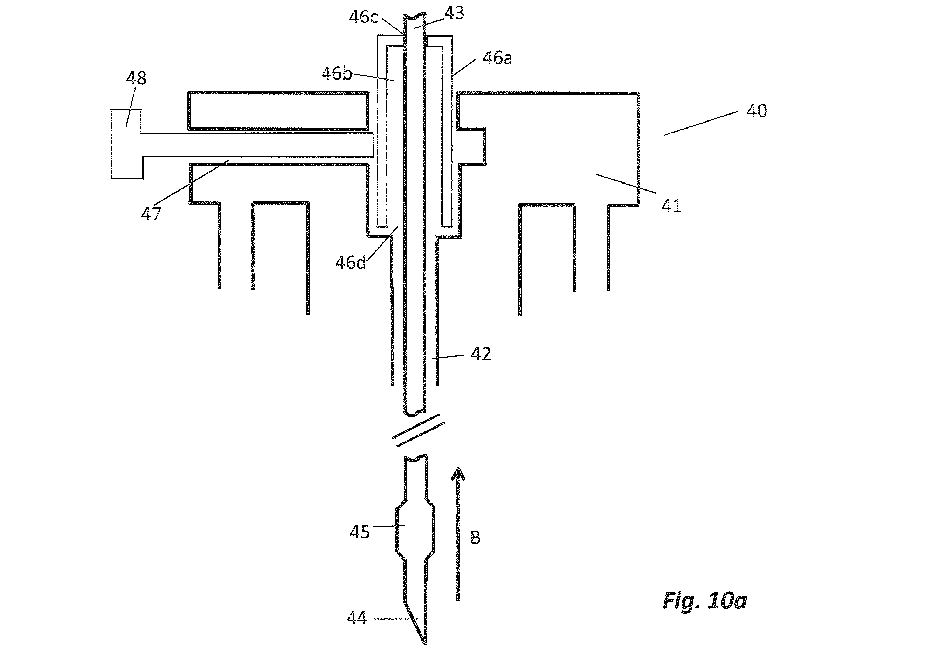

Figure 10a shows a cross section of a male luer connector 40 in which a puncture needle 43 is inserted. The male luer connector 40 may for example be connected to a catheter hub as disclosed herein, e.g. as shown in Fig. 7. The catheter hub is however not shown in this Figure. The puncture needle 43 extends in the through hole 42 of the male luer connector 40. The through hole 42 ends at the base portion 41 with a hollow casing 46a. Thus, the hollow casing is positioned at the proximal end of the male luer connector if the connector is connected to a catheter hub. The base portion 41 also has a coring-out channel 47 that extends substantially perpendicular to the through hole 42 to the outside of the base portion 41. A shutter 48 is spring loaded in the coring out channel 47 by a rubber band (not shown) extending around the base portion 41. The shutter may thus be mounted as the shutter seen in Fig. 8. The shutter abuts the hollow casing 46a, and consequently does not abut the surface of the needle 43.

Close to the tip 44 of the needle 43, such as about 1 cm from the tip 44 of the needle 43, the needle 43 has been flattened in one direction to form a locking portion 45. The locking portion has a diameter that is larger than the average diameter of the needle extending through the luer connector 40 but is still slidable in the through hole 42, i.e. the through hole 42 has a larger diameter than the locking portion 45. As indicated in Fig. 10a, the tip of the needle extends out on the other side of the male luer connector 40. If for example the connector 40 is attached to a catheter hub as shown in Fig. 7, the tip 44 as well as the locking portion 45 may be positioned on the distal end of the catheter hub.

The hollow casing 46a comprises an inlet 46c, a hollow interior 46b and an outlet 46d, and the puncture needle extends through the interior 46b and out of both the inlet 46c and the outlet 46d of the hollow casing 46a. The diameter of the outlet 46d is larger than the diameter of the inlet 46c. Further, the diameter of the inlet 46c, which thus is positioned at the proximal end if the connector 40 is attached to a catheter hub, has a smaller diameter compared to the locking portion 45 of the needle 43. Thus, as seen in Fig. 10a, the hollow interior 46b of the casing 46a forms a part, or an extension, of the through hole 42 of the male luer connector 40.

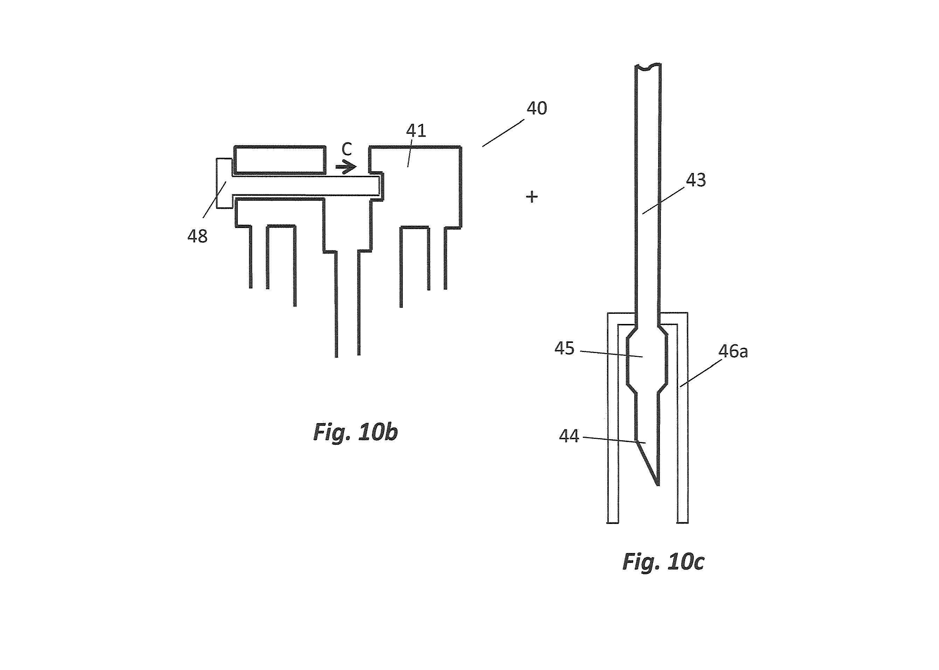

Upon removal of the needle 43 from the connector 40 in the direction as indicated by arrow B, i.e. in the direction from the female luer connector to the "outside" of the male luer connector 40 if connected to a female luer connector as e.g. shown in Fig. 7, the locking portion 45 of the needle 43 slides through the through hole 42 and into the hollow casing 46a via outlet 46d, but cannot be withdrawn through the inlet 46c of the hollow casing 46a due to the smaller diameter of the inlet 46c compared to the locking portion 45. This means that the whole hollow casing 46a, which is removably connected to the base portion 41 of the male luer connector 40, is withdrawn together with the needle 43 from the male luer connector 40, as seen in Fig. 10c. When the needle 43 has been removed, the tip 44 is protected by hollow casing 46a.

Upon removal of the needle 43 and thereby also the hollow casing 46a, the spring loaded shutter 48 may automatically seal the through hole 42 by moving in the coring-out channel 47 in the direction indicated by arrow C in Fig. 10b. Thus, the male luer connector as schematically seen in Fig. 10a-c both provides for automatic sealing of the through hole of the male luer connector and for tip protection upon removal of a needle from the connector.

In summary, the present invention simplifies the insertion of peripheral intravenous catheter by already having a male luer connector (a luer "cap") connected to the needle holder, omitting the procedure of applying a separate luer cap. Blood spill from the catheter is further eliminated at insertion, thereby reducing the risk for blood mediated infections. A double valve concept further reduces the risk for external contamination/air to the inside of the catheter hub or vasculature.

## Claims

1. A male luer connector for sealing a female luer connector, said male luer connector comprising
- a base portion adapted to removably connect with the female luer connector;
- a protruding portion adapted to be inserted into the female luer connector;
- a through hole extending through the protruding portion and the base portion and adapted to receive a needle; and
- a valve device arranged in said base portion and adapted to block fluid flowing in said through hole in the direction from the female luer connector via the protruding portion out of the male luer connector when the male luer connector is connected to the female luer connector.

2. A male luer connector according to claim 1, wherein the valve device is adapted to receive the needle in a substantially frictionless manner and/or adapted to allow a received needle to slide in said through hole in a substantially frictionless manner.

3. A male luer connector according to claim 1 or 2, wherein the valve device is adapted to seal against the needle when a needle is inserted in the through hole.

4. A male luer connector according to any previous claim, wherein the valve device is a one way check valve, such as a duck-bill check valve.

5. A male luer connector according to claim 4, further comprising a dome slit valve arranged in said through passage with the convex surface facing the exterior when the male luer connector is connected to said female luer connector.

6. A male luer connector according to any one of claims 1-3, wherein the valve device is a dome slit valve arranged in said through passage with the convex surface facing the exterior when the male luer connector is connected to said female luer connector.

7. A male luer connector according to claim 6, further comprising a second dome slit valve arranged in said through passage with the convex surface facing female luer connector when the male luer connector is connected to said female luer connector.

8. A male luer connector according to any one of claims 5-7, wherein the slit edges of the dome slit valve extend towards the center of the dome, thereby forming a tight passage extending from the surface of the dome towards the center of the dome.

9. A male luer connector according to claim 8, wherein at least one dome slit valve comprises at least one slit edge reinforcement means arranged on the concave side of the dome, said reinforcement means extending substantially perpendicular to the direction of the slit from at least one extended slit edge to the concave surface of the dome, for increasing the pressure the extended slit edges may withhold before opening.

10. A male luer connector according to claim 1 or 2, wherein the valve device is a shutter arranged in a coring-out channel of said base portion, wherein said channel extends from said through hole towards the outside of the luer cap, and wherein said shutter is arranged to seal said through hole in the absence of said needle.

11. A male luer connector according to claim 10, wherein said shutter is adapted to be spring loaded in said channel by said needle such that the shutter automatically seals said through hole in the absence of said needle.

12. A male luer connector according to claim 10 or 11, wherein the through hole further comprises a removable inner tubing or casing in the base portion such that the shutter in the coring-out channel is prevented from abutting a puncture needle present in the through hole.

13. A male luer connector according to any previous claim, further comprising a cover to prevent contamination from the outside into said through hole when the male luer connector when connected to the female luer connector.

14. Use of a luer connector according to any one of claims 1-13 in a catheter hub for restricting back flow of blood from the catheter to the exterior upon removal of a puncture needle from the catheter hub, wherein said luer connector is removably connected to a proximal female luer connector of the catheter hub and wherein said needle is the needle used to insert the catheter into the vascular system of a patient and is removed from the vascular system and said catheter hub through said through hole of said luer connector.

15. A catheter hub comprising
- a catheter at a distal end,
- a female luer connector at a proximal end,
- a male luer connector according to any one of claims 1-13, removably connected to said female luer connector, and
- a puncture needle extending through said catheter, said catheter hub and said through hole of said luer connector.

16. A catheter hub according to claim 15, in which the valve device of the male luer connector is a shutter according to any one of claims 10-12, and wherein
said through hole of said male luer connector ends with a removable hollow casing for said needle in said base portion, said casing comprising an inlet facing the outside of the male luer connector and an outlet facing the female luer connector when the male luer connector is connected to the female luer connector, and a hollow interior between said inlet and outlet through which the needle extends, wherein the hollow casing is arranged such that the shutter abuts the casing and not the needle,
and wherein said needle comprises a lock portion arranged between the tip and the catheter hub, said lock portion having a larger diameter in at least one direction compared to the diameter of the part of the needle extending through the catheter hub and the male luer connector,
and wherein the diameter of the through hole of the male luer connector, the diameter of said outlet of the casing and the diameter of the hollow interior of the casing are larger than the diameter of said lock portion of said needle, and wherein the diameter of said inlet of the hollow casing is smaller than the diameter of said lock portion of said needle such that said lock portion and the tip of the needle get stuck in said hollow casing upon removal of the needle from the catheter hub in the direction from the female luer connector to the male luer connector, such that the hollow casing may be withdrawn from the male luer connector together with the needle.

17. A catheter hub according to claim 15 or 16, further comprising a needle holder removably connected to the male luer connector.

18. A catheter hub according to claim 17, wherein the external needle holder comprises a valve device for blocking fluid flowing from said needle holder into said through hole of said male luer connector.

19. A method for inserting a catheter in the vascular system of a patient, comprising the steps of:
a) providing a catheter hub according to any one of claims 15-18,
b) puncturing a vessel of the vascular system of the patient with the puncture needle and pushing said catheter over the puncture needle into said vessel; and
c) pulling said puncture needle out from the vascular system and said catheter hub via the male luer connector.

20. A method according to claim 19, further comprising the step of
d) putting a cover on the male luer connector for preventing the risk of external contamination into said catheter hub.
